# EUROPÄISCHE PATENTSCHRIFT

(11) **EP 0 436 091 B1**
(45) Veröffentlichungstag und Bekanntmachung des Hinweises auf die Patenterteilung: **25.01.1995**
(21) Anmeldenummer: 90121663.0
(22) Anmeldetag: 13.11.1990
(51) Int. Cl.: A61B 1/32, A61B 17/42

(54) **Instrumenten-Satz für Eingriffe am Uterus (*)**
Set of instruments for operations on the uterus
Jeu d'instruments pour interventions chirurgicales de l'utérus

(30) Priorität: 04.12.1989 DE 3940064
(43) Veröffentlichungstag der Anmeldung: 10.07.1991
(60) Teilanmeldung: 91118952.0
(73) Patentinhaber: LABOTECT-LABOR-TECHNIK, GÖTTINGEN, GMBH, D-37120 Bovenden (DE)
(72) Erfinder: Schinkel, Otto, W-3406 Bovenden (DE); Horvath, Josef, W-3400 Göttingen (DE)
(74) Vertreter: Rehberg, Elmar, Dipl.-Ing.

(56) Entgegenhaltungen:
- FR-A- 820 618
- FR-A- 2 635 453
- US-A- 2 844 144
- US-A- 4 432 352

## Beschreibung

Die Erfindung bezieht sich auf einen Instrumenten-Satz für Eingriffe am Uterus mit den Merkmalen des Oberbegriffs des Anspruchs 1. Ein solcher Instrumenten-Satz erleichtert dem Gynäkologen die Handhabung, wenn er operative Eingriffe am Uterus vorzunehmen hat. Der Instrumenten-Satz kann aber darüberhinaus auch zum transvaginalen Gametentransfer und zur Tubenkatheterisierung eingesetzt werden.

Ein Instrumenten-Satz nach dem Oberbegriff des Anspruchs 1 ist aus der US-A-28 44 144 bekannt. Das zugehörige Spekulum weist einen glatten Oberlöffel und einen glatten Unterlöffel auf. Dabei ist der Unterlöffel nicht direkt, sondern über einen zusätzlichen Schwenkarm an dem Oberlöffel gelagert. Der Unterlöffel ist gegenüber dem Schwenkarm derart verschiebbar, daß eine zweite Spreizeinrichtung des Spekulums gebildet wird. Die Kugelzange des bekannten Instrumenten-Satzes weist in ihrem proximalen Bereich an einem ihrer Arme eine längs des Arms verschiebbare und arretierbare Befestigungsplatte mit einem randoffenen Schlitz auf. Die Befestigungsplatte ist mit einer den randoffenen Schlitz in Querrichtung durchgreifenden Sicherungsschraube an eine Gegenplatte eines Widerlagers an dem Spekulum anschraubbar. Dabei erlaubt der randoffene Schlitz ein Einsetzen der Befestigungsplatte in das Widerlager, ohne daß die Sicherungsschraube vollständig gelöst werden muß.

Ein Instrumenten-Satz mit einem Spekulum und einer Kugelzange ist auch aus der FR-A-820 618 bekannt. Das Spekulum weist zwei über Querstangen miteinander verbundene seitliche Löffel auf. Das Spreizen der Löffel erfolgt in gerader Richtung voneinander weg entlang der Querstangen. Beide Löffel sind glatt und geschlossen ausgebildet. Die Kugelzange weist eine Befestigungsplatte mit verschiedenen randoffenen Schlitzen auf. Die Kugelzange ist über die verschiedenen Schlitze wahlweise an eine Querstange des Spekulums anhängbar. Auf der der Befestigungsplatte abgekehrten Seite weist die Kugelzange eine Klemmvorrichtung zur Aufnahme eines Führungsrohrs auf.

Es ist noch ein weiterer Instrumenten-Satz für Eingriffe am Uterus mit einem Spekulum, welches einen Oberlöffel und einen daran mit einer Spreizeinrichtung schwenkbar gelagerten Unterlöffel aufweist, und mit einer Kugelzange zum Ergreifen der Portio bekannt, bei dem der Ober- und der Unterlöffel, die insgesamt einen etwa rohrförmigen Umriß umschließen, glattflächig ausgebildet sind. Der Oberlöffel weist in seinem proximalen Teil fast exakt Rohrform auf. In den seitlichen Wangen ist der Unterlöffel schwenkbar gelagert. Die beiden Löffel sind an ihrem proximalen Teil zu Spreizhebeln verlängert, die zu einer Spreizeinrichtung gehören, welche ansonsten noch eine Schraube und eine Mutter aufweist. Mit dieser Spreizeinrichtung können der Oberlöffel und der Unterlöffel nach ihrer Einführung in die Scheide und nach entsprechender Drehung gegeneinander gespreizt werden, so daß dem Arzt die Draufsicht auf die Portio ermöglicht ist. Zu diesem bekannten Instrumenten-Satz gehört eine Kugelzange, die zum Ergreifen der Portio und zum Vorziehen des Uterus dient. Die Kugelzange weist von ihrem Gelenk nach hinten gerade abstehende Griffenden auf und ist an ihrem distalen Ende mit abgewinkelten Hebelenden versehen. Nachteilig ist an diesem Instrumenten-Satz, daß infolge der glattflächigen Ausbildung der Löffel die Gefahr besteht, daß das Spekulum aus der Scheide herausrutscht, wenn es nicht kontinuierlich festgehalten wird. Außerdem muß die Kugelzange, nachdem mit ihr die Portio ergriffen wurde, ebenfalls festgehalten werden. Somit werden zum Festhalten des Spekulums und der Kugelzange schon zwei Hände benötigt und der Arzt muß sich diesbezüglich einer Hilfe bedienen, weil er sonst keine Hand mehr frei hätte, um den operativen Eingriff auszuführen. Für den transvaginalen Gametentransfer wird der Instrumenten-Satz um einen Kunststoffkatheter ergänzt.

Der Erfindung liegt die Aufgabe zugrunde, einen Instrumenten-Satz der eingangs beschriebenen Art so weiterzubilden, daß das Spekulum und die Kugelzange angewendet werden können, ohne daß hierzu ständig zwei Hände benötigt werden. Der Arzt soll für den operativen Eingriff die Hände freibekommen. In Weiterbildung soll der Instrumenten-Satz auch zum transvaginalen Gametentransfer und zur Tubenkatheterisierung besser geeignet sein.

Erfindungsgemäß wird dies durch Verreicht, die kennzeichnenden Merkmale des Anspruchs 1. Infolge der Aussparungen im Ober- und im Unterlöffel tritt nach dem Einsetzen des Spekulums und dem Spreizen mit Hilfe der Spreizeinrichtung die muskulöse Scheidenwandung teilweise in diese Aussparungen ein, so daß ein fester Sitz erreicht wird und auch Preßbewegungen nicht zu einem Herausrutschen des Spekulums aus der Scheide führen können. Das Widerlager am Spekulum dient zur Aufnahme eines korrespondierenden Fortsatzes an der Kugelzange, nachdem mit der Kugelzange die Portio ergriffen und der Uterus in eine möglichst gestreckte Lage überführt worden ist. Nachdem die Kugelzange mit dem Fortsatz in dem Widerlager des Spekulums eingehängt ist, befinden sich diese, Teile des Instrumenten-Satzes in ihrer ordnungsgemäßen Relativlage. Sie müssen nicht mehr von Hand festgehalten werden, sondern der Arzt hat beide Hände frei, um den operativen Eingriff durchzuführen. Der Oberlöffel ist in aller Regel etwas kürzer ausgebildet als der Unterlöffel, und zwar an den distalen Enden, wodurch der Uterus in eine günstigere Lage kommt.

Der Oberlöffel kann zweckmäßig an seinem proximalen Ende eine randoffene Ausnehmung zur Vergrößerung der freien Draufsicht auf die Portio aufweisen. Diese randoffene Ausnehmung ist bis in den Bereich der Seitenwangen des Oberlöffels geführt und erstreckt sich in der Tiefe auf etwa 1/4 der Länge des Oberlöffels.

Die Kugelzange kann im Bereich zwischen ihrem Gelenk und den freien Griffenden der Gelenkarme abgewinkelt ausgebildet sein. Diese Abwinklung beträgt etwa 45°, so daß die freien Griffenden aus dem Arbeitsbereich des Operateurs hinaus verlagert werden.

Der Fortsatz an der Kugelzange ist unterhalb des Gelenks vorgesehen und weist eine Führungsplatte auf, die von einem beidseitig auskragenden Bolzen durchsetzt ist; das Widerlager an dem Spekulum weist zwei Führungswände auf, die in größerem Abstand als die Dicke der Führungsplatte angeordnet und mit den randoffenen Schlitzen versehen sind. Dies ist eine besonders vorteilhafte Ausführungsform, weil dadurch die Kugelzange nicht nur einhängbar festgehalten wird, sondern zugleich auch geführt und gegen Verdrehung gesichert ist. Andere Ausführungsformen sind natürlich auch denkbar.

Mit besonderem Vorteil kann die Kugelzange auf der dem Fortsatz abgekehrten Seite eine Klemmhalterung mit federnd gelagerten Klemmbacken zum Aufnehmen eines Katheterführungsrohrs aufweisen. Damit wird es möglich, das Katheterführungsrohr, welches das Einführen des Katheters erleichtert, in der Klemmhalterung der Kugelzange festzusetzen, wobei die Kugelzange an dem Spekulum eingehängt ist. Insoweit erhält auch das Katheterführungsrohr nach seinem Einführen den erforderlichen festen Sitz in seinem proximalen Endbereich, so daß es nicht mit der Hand bei der Handhabung des Katheters festgehalten werden muß.

Die Erfindung wird anhand eines bevorzugten Ausführungsbeispiels weiter erläutert und beschrieben. Es zeigen:
- Figur 1: eine teilweise geschnittene Darstellung wesentlicher Teile des Instrumenten-Satzes in der Gebrauchslage,
- Figur 2: eine Ansicht von Teilen des Instrumenten-Satzes in einzelner Darstellung und
- Figur 3: eine Draufsicht auf das Spekulum von oben.

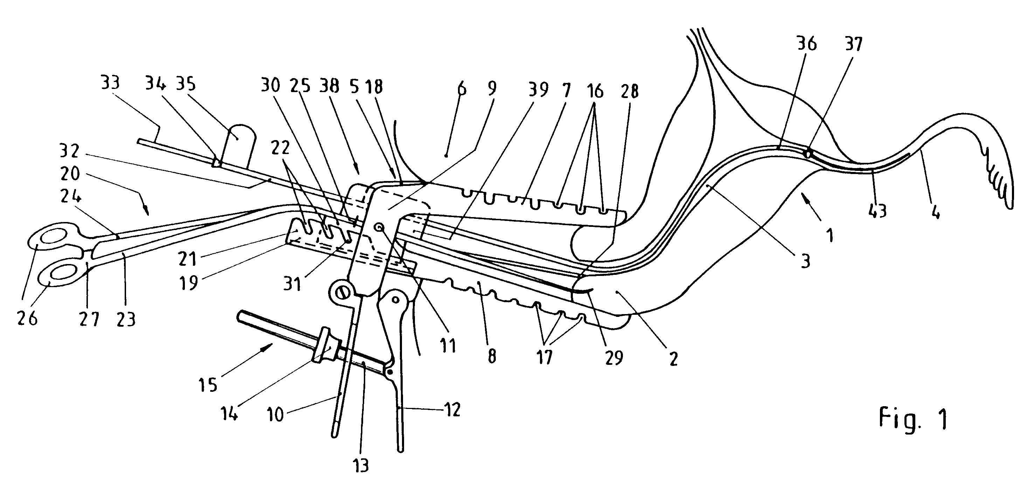

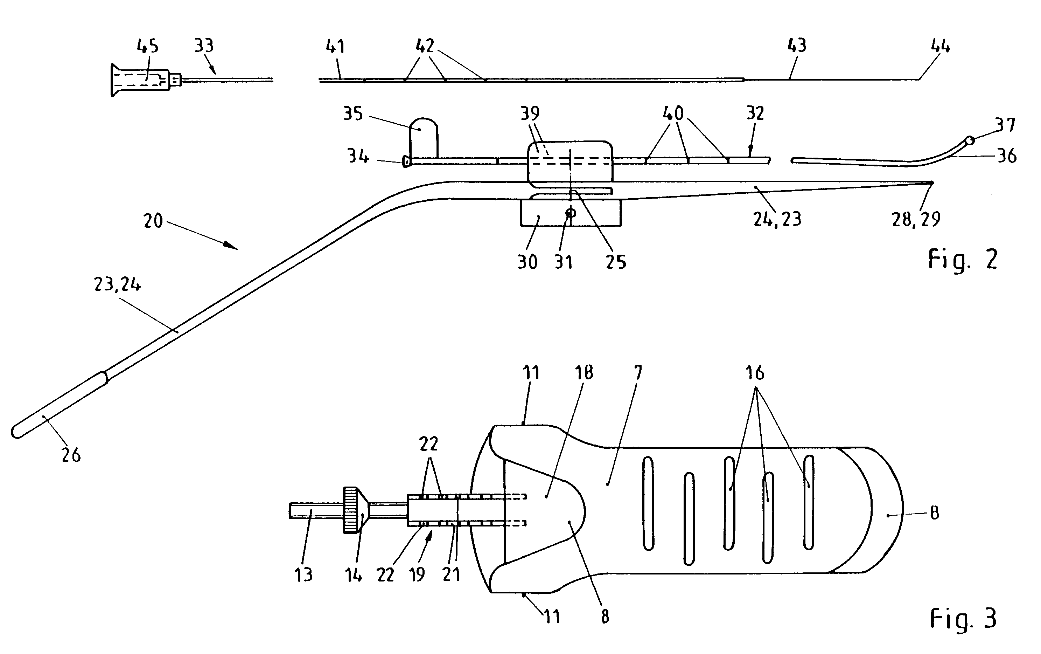

In Figur 1 ist schematisch und teilweise ein Uterus 1 mit Portio 2, Endometrium 3 und einer Tube 4 dargestellt. Ein Spekulum 5 ist in eine Scheide 6 eingeführt. Das Spekulum 5 weist einen Oberlöffel 7 und einen Unterlöffel 8 auf. Der Oberlöffel 7 ist an seinem proximalen Teil über zwei Seitenwangen 9 fast rohrförmig ausgebildet. Vorne am unteren Ende greift ein Spreizhebel 10 an. Der Oberlöffel 7 weist etwa im Mittelbereich der Seitenwangen 9 ein Gelenk 11 auf, mit dessen Hilfe der Unterlöffel 8 an seinem proximalen Teil schwenkbar an dem Oberlöffel 7 gelagert ist. Der Unterlöffel 8 setzt sich in einem Spreizhebel 12 fort. An ihm ist eine Schraube 13 gelenkig angelenkt, die durch eine Ausnehmung im Spreizhebel 10 hindurchreicht. Der Schraube 13 ist eine Mutter 14 zugeordnet, so daß auf diese Art und Weise durch Verdrehen der Mutter 14, die dann an dem Spreizhebel 10 anliegt, insgesamt eine Spreizeinrichtung 15 geschaffen ist, mit deren Hilfe durch relatives Verdrehen der Mutter 14 Oberlöffel 7 und Unterlöffel 8 des Spekulums 5 gegeneinander gespreizt bzw. zusammengeführt werden können. Das Spekulum 5 ist in Figur 1 in der gewünschten gespreizten Lage dargestellt. Es wird in ungespreiztem Zustand in die Scheide 6 eingeführt, um 90° gedreht und dann mit der Spreizeinrichtung 15 gespreizt. Der Oberlöffel 7 weist Aussparungen 16 und der Unterlöffel 8 Aussparungen 17 auf. Diese Aussparungen 16, 17 sind randgeschlossen ausgebildet und haben die Form von langlochartigen Schlitzen, die zudem noch versetzt zueinander (Figur 3) angeordnet sind. Die Aussparungen 16 und 17 können auch andere Form besitzen, beispielsweise länglich oder kreisrund ausgebildet sein. Beim Spreizen des Spekulums 5 legt sich auf diese Art und Weise die Wandung der Scheide 6 teilweise in die Aussparungen 16 und 17 hinein, wodurch das Spekulum seinen festen, unverrutschbaren Sitz erhält. Der Oberlöffel 7 weist an seinem proximalen Teil eine randoffene Ausnehmung 18 auf, siehe auch Figur 3, wodurch der Einblicksbereich des Arztes auf die Portio 2 wesentlich verbessert wird.

An dem Spekulum 5, insbesondere an dem Unterlöffel 8, ist ein Widerlager 19 für eine Kugelzange 20 ausgebildet und vorgesehen. Das Widerlager 19 kann U-förmigen Querschnitt aufweisen, so daß auf diese Art und Weise zwei Führungswände 21 gebildet sind, die parallel zueinander angeordnet sind und einen Abstand zueinander aufweisen. Diese Führungswände 21 sind mit randoffenen Schlitzen 22 versehen, die, wie aus Figur 1 ersichtlich, schräg angeordnet sind.

Die Kugelzange 20 weist zwei Gelenkarme 23 und 24 auf, die nach Art einer Schere über ein Gelenk 25 miteinander in Verbindung stehen. Am proximalen Ende der Gelenkarme 23 und 24 befinden sich Griffe 26. Zwischen diesen ist eine Feststellbrücke 27 angeordnet, wie sie auch bei anderen ärztlichen Instrumenten bekannt ist. Die Gelenkarme 23 und 24 sind etwa im Bereich zwischen dem Gelenk 25 und den Griffen 26 abgewinkelt, wobei die Abwinklung etwa um 45° nach unten verläuft. Die proximalen Enden der Gelenkarme 23 und 24 weisen nach innen gerichtete Umbiegungen 28 und 29 auf, mit denen die Portio 2 ergriffen und festgehalten werden kann.

Die Kugelzange 20 weist darüberhinaus etwa unterhalb des Bereichs, in welchem das Gelenk 25 angeordnet ist, eine Führungsplatte 30 auf, die beispielsweise unter den Gelenkarm 24 geschraubt ist. Diese Führungsplatte 30 weist eine kleinere Dicke auf, als es dem Abstand der Führungswände 21 des Widerlagers 19 voneinander entspricht, so daß die Kugelzange 20 in dem Widerlager 19 geführt eingesetzt werden kann. Die Führungsplatte 30 wird von einem Bolzen 31 durchsetzt, der rechts und links aus der Führungsplatte 30 um einen gewissen Betrag übersteht, und zwar derart, daß er in Wirkverbindung mit den Schlitzen 22 in den Führungswänden 21 treten kann.

Ein solcher Instrumenten-Satz wird wie folgt gehandhabt: Zunächst wird das Spekulum 5 in die ungespreizte Stellung überführt, also durch Verdrehen der Mutter 14 relativ zur Schraube 13 eine Lage geschaffen, in welcher sich Oberlöffel 7 und Unterlöffel 8 sehr nahekommen bzw. einander berühren. In dieser Stellung wird das Spekulum 5 in die Scheide 6 eingeführt und um etwa 90° gedreht. Anschließend erfolgt das Spreizen mit Hilfe der Spreizeinrichtung 15, wodurch sich über das Gelenk 11 Oberlöffel 7 und Unterlöffel 8 voneinander entfernen, bis ein fester, unverrutschbarer Sitz des Spekulums 5 in der Scheide 6 erreicht ist. Anschließend wird die Portio 2 mit Hilfe der Kugelzange 20 ergriffen und der Uterus 1 durch Ziehen an der Kugelzange 20 in eine weitgehend gestrecke Lage gebracht. In dieser Stellung wird die Kugelzange 20 mit Hilfe des Bolzens 31 in einen Schlitz 22 des Widerlagers 19 eingehängt, so daß nunmehr der Arzt beide Hände frei hat, um den operativen Eingriff vorzunehmen. Dabei ermöglicht die Ausnehmung 18 im Oberlöffel 7 eine weitgehend ungehinderte Draufsicht auf die Portio 2.

Zum transvaginalen Gametentransfer und zur Tubenkatheterisierung wird der Instrumenten-Satz um ein Katheterführungsrohr 32 und einen Katheter 33 ergänzt. Das Katheterführungsrohr 32 weist an seinem proximalen Ende einen Einführkonus 34 für den Katheter 33 auf. In diesem Endbereich ist auch ein radial abgesetzter Flügel 35 vorgesehen, der so angeordnet ist, daß er mit einer vorgeformten Krümmung 36 im distalen Endbereich des Katheterführungsrohrs 32 übereinstimmt. Direkt am distalen Ende des Katheterführungsrohrs 32 ist ein Kugelstück 37 vorgesehen.

Während unterhalb des Gelenks 25 der Kugelzange 20 die Führungsplatte 30 vorgesehen ist, ist oberhalb dieses Gelenks 25 eine Klemmhalterung 38 aus zwei federnd gegeneinander angepreßt gelagerten Klemmbacken 39 vorgesehen, wobei zwischen den beiden Klemmbacken 39 ein Klemmspalt gebildet wird, der in seiner Ausbildung und Abmessung auf die Aufnahme des Katheterführungsrohrs 32 abgestimmt ist, so daß nach dem Setzen des Katheterführungsrohrs 32 dieses zwischen die Klemmbacken 39 der Klemmhalterung 38 eingeklemmt werden kann, wodurch es ebenfalls einen gesicherten Sitz erhält und letztlich über die Kugelzange 20 an dem Spekulum 5 gelagert ist. Außen auf dem Katheterführungsrohr 32 ist eine Skala 40 angebracht, die mit den Klemmbacken 39 zusammenarbeitet und der Orientierung dient.

Der Katheter 33 besteht im wesentlichen aus einem dünnen Kunststoffrohr 41 mit einer Skala 42, und einem distalen Endteil 43, welches gegenüber dem übrigen Teil des Kunststoffrohrs 41 einen reduzierten Durchmesser aufweist. Das distale Ende 44 des Katheters 33 ist abgerundet ausgebildet. Am proximalen Ende des Katheters 33 ist ein Anschluß 45 für eine Spritze vorgesehen.

Die Benutzung des Instrumenten-Satzes aus Spekulum 5, Kugelzange 20, Katheterführungsrohr 32 und Katheter 33 geschieht wie folgt:
Zunächst werden das Spekulum 5 und die Kugelzange 20 so gehandhabt wie vorher beschrieben. Es erfolgt sodann das Einführen des Katheterführungsrohrs 32. Hat das Katheterführungsrohr 32 seinen ordnungsgemäßen Sitz erreicht, so wird es in die Klemmhalterung 38 eingeklemmt, wobei sich der Arzt anhand der Skala 40 die ordnungsgemäße Lage und damit die Einführtiefe merken kann, was für einen Zweit- oder Mehrfacheinsatz des Instrumenten-Satzes für die gleiche Patientin sinnvoll ist. Nachdem das Katheterführungsrohr 32 auf diese Art und Weise in der Klemmhalterung 38 zwischen den Klemmbacken 39 eingeklemmt und damit gehalten ist, werden wiederum beide Hände des Arztes frei für die Handhabung des Katheters 33. Dieser wird in das Katheterführungsrohr 32 eingeführt. Anschließend kann der transvaginale Gametentransfer erfolgen.

### Bezugszeichenliste:

- 1: = Uterus
- 2: = Portio
- 3: = Endometrium
- 4: = Tube
- 5: = Spekulum
- 6: = Scheide
- 7: = Oberlöffel
- 8: = Unterlöffel
- 9: = Seitenwange
- 10: = Spreizhebel
- 11: = Gelenk
- 12: = Spreizhebel
- 13: = Schraube
- 14: = Mutter
- 15: = Spreizeinrichtung
- 16: = Aussparungen
- 17: = Aussparungen
- 18: = Ausnehmung
- 19: = Widerlage
- 20: = Kugelzange
- 21: = Führungswand
- 22: = Schlitz
- 23: = Gelenkarm
- 24: = Gelenkarm
- 25: = Gelenk
- 26: = Griff
- 27: = Feststellbrücke
- 28: = Umbiegung
- 29: = Umbiegung
- 30: = Führungsplatte
- 31: = Bolzen
- 32: = Kathetherführungsrohr
- 33: = Katheter
- 34: = Einführkonus
- 35: = Flügel
- 36: = Krümmung
- 37: = Kugelstück
- 38: = Klemmhalterung
- 39: = Klemmbacke
- 40: = Skala
- 41: = Kunststoffrohr
- 42: = Skala
- 43: = Endteil
- 44: = Ende
- 45: = Anschluß

## Patentansprüche

1. Instrumenten-Satz für Eingriffe am Uterus (1) mit einem Spekulum (5), welches einen Oberlöffel (7) und einen daran mit einer Spreizeinrichtung (15) schwenkbar gelagerten Unterlöffel (8) aufweist, und mit einer Kugelzange (20) zum Ergreifen der Portio (2) durch die zentrale Öffnung des Spekulums (5), wobei an der Kugelzange (20) ein Fortsatz (30, 31) vorgesehen ist, so daß die Kugelzange (20) mittels eines randoffenen Schlitzes (22) in ein Widerlager (19) an dem Spekulum (5) einsetzbar ist, dadurch gekennzeichnet, daß im Ober- und im Unterlöffel (7, 8) des Spekulums (5) Aussparungen (16, 17) für einen festen Sitz des Spekulums in der Scheide (6) vorgesehen sind und daß das Widerlager (19) an dem Spekulum (5) mit verschiedenen randoffenen Schlitzen (22) versehen ist, in die der Fortsatz der Kugelzange wahlweise einhängbar ist.

2. Instrumenten-Satz nach Anspruch 1, dadurch gekennzeichnet, daß der Oberlöffel (7) an seinem proximalen Ende eine randoffene Ausnehmung (18) zur Vergrößerung der freien Draufsicht auf die Portio (2) aufweist.

3. Instrumenten-Satz nach Anspruch 1, dadurch gekennzeichnet, daß die Kugelzange (20) im Bereich zwischen ihrem Gelenk (25) und den freien Griffenden (26) der Gelenkarme (23, 24) abgewinkelt ausgebildet ist.

4. Instrumenten-Satz nach Anspruch 1, dadurch gekennzeichnet, daß der Fortsatz (30, 31) an der Kugelzange (20) unterhalb ihres Gelenks (25) vorgesehen ist und eine Führungsplatte (30) aufweist, die von einem beidseitig auskragenden Bolzen (31) durchsetzt ist, und daß das Widerlager (19) an dem Spekulum (5) im unteren Bereich dessen zentraler Öffnung angeordnet ist und zwei Führungswände (21) aufweist, die in größerem Abstand als die Dicke der Führungsplatte (30) angeordnet sowie mit den randoffenen Schlitzen (22) versehen sind.

5. Instrumenten-Satz nach einem der Ansprüche 1 bis 4, dadurch gekennzeichnet, daß die Kugelzange (20) auf der dem Fortsatz (30, 31) abgekehrten Seite eine Klemmhalterung (38) mit federnd gelagerten Klemmbacken (39) zum Aufnehmen eines Katheterführungsrohrs (32) aufweist.

## Claims

1. Instrument set for surgical operations on the uterus (1) having a speculum (5), which comprises an upper spoon (7) and a lower spoon (8) which is pivotably mounted therein with a spreading device (15), and forceps (20) for the purpose of engaging the portio (2) through the central opening of the speculum (5), wherein an extension piece (30, 31) is provided at the forceps (20) in such a manner that the forceps (20) can be inserted by means of an edge-open slot (22) into a counter bearing (19) at the speculum (5), characterized in that in the upper spoon (7) and the lower spoon (8) of the speculum (5) recesses are provided (16, 17) for a firm fit of the speculum in the vagina (6) and that the counter bearing (19) at the speculum (5) is provided with various edge-open slots (22), into which the extension piece of the forceps can be optionally suspended.

2. Instrument set according to claim 1, characterized in that the upper spoon (7) comprises at its proximal end an edge-open recess (18) for the purpose of enlarging the free plan view of the portio (2).

3. Instrument set according to claim 1, characterized in that the forceps (20) are curved in the region between their joint (25) and the free grip end (26) of the articulated arms (23, 24).

4. Instrument set according to claim 1, characterized in that the extension piece (30, 31) is provided at the forceps (20) below their joint (25) and comprises a guide plate (30), which is penetrated by a pin (31) which overhangs at both sides, and that the counter bearing (19) is disposed at the speculum (5) in the lower region of the central opening thereof and comprises two guide walls (21), which are disposed at a greater distance than the thickness of the guide plate (30) and are provided with the edge-open slots (22).

5. Instrument set according to any one of claims 1 to 4, characterised in that the forceps (20) comprise on the side remote from the extension piece (30, 31) a clamping holder (38) having resiliently mounted clamping cheeks (39) for the purpose of receiving a catheter guide tube (32).

## Revendications

1. Jeu d'instruments pour des interventions sur l'utérus (1), avec un spéculum (5) qui présente une cuillère supérieure (7) cet une cuillère inférieure (8) montée pivotante sur celle-ci avec un dispositif écarteur (15), et avec une pince (20) a portée sphérique pour saisir le col (2) à travers l'ouverture centrale du spéculum (5), un prolongement (30, 31) étant prévu sur la pince (20), de telle sorte que la pince (20) peut être insérée dans un contrefort (19) sur le spéculum (5) au moyen d'une encoche (22) à bord ouvert,
**caractérisé** en ce que des ouvertures (16, 17) sont prévues dans la cuillère supérieure et dans la cuillère inférieure (7, 8) pour une assise ferme du spéculum (5) dans le vagin (6) et en ce que le contrefort (19) sur le spéculum est muni de diverses encoches (22) à bord ouvert, dans lesquelles le prolongement de la pince à portée sphérique peut être accroché sélectivement.

2. Jeu d'instruments selon la revendication 1, caractérisé en ce que la cuillère supérieure (7) présente sur son extrémité proximale une ouverture (18) à bord ouvert pour agrandir le champ de vision libre vers le col (2).

3. Jeu d'instruments selon la revendication 1, caractérisé en ce que la pince (20) à portée sphérique a une forme coudée dans la région entre son articulation (25) et les extrémités libres, munies de poignées, (26) des branches articulées (23, 24)

4. Jeu d'instruments selon la revendication 1, caractérisé en ce que le prolongement (30, 31) sur la pince (20) à portée sphérique est prévu en dessous de son articulation (25) et présente une plaque de guidage (30) qui est traversée par un goujon (31) dépassant des deux côtés, et en ce que le contrefort (19) est disposé sur le spéculum (5) dans la région inférieure de son ouverture centrale et présente deux parois de guidage (21) qui sont disposées à un espacement plus grand que l'épaisseur de la plaque de guidage (30) et sont munies des encoches (22) à bord ouvert.

5. Jeu d'instruments selon une des revendications 1 à 4, caractérisé en ce que la pince (20) à portée sphérique présente sur le côté opposé au prolongement (30, 31) un support de serrage (38) avec des mâchoires (39) montées élastiquement pour recevoir un tube (32) de guidage d'un cathéter.
